# EUROPEAN PATENT APPLICATION

(11) **EP 1 709 919 A1**
(43) Date of publication of application: **11.10.2006**
(21) Application number: 06388024.9
(22) Date of filing: 31.03.2006
(51) Int. Cl.: A61B 17/34

(54) **Needle guide with positioning means**

(30) Priority: 05.04.2005 DK 200500469
(71) Applicant: B-K Medical ApS, 2730 Herlev (DK)
(72) Inventor: Sasady, Niels Chr., 4700 Naestved (DK); Pedersen, Morten Høholm, 4700 Naestved (DK)
(74) Representative: Siiger, Joergen

(57) **Abstract**

A needle guide supporting device with clips means (2) for detachable fastening to an ultrasound head (3), said device being shaped for detachable reception of a wedge-shaped needle guide (5). According to the invention, the device is provided with a rotatable handle (4) being adjustable and optionally fixable in various positions for the insertion and fastening of said needle guide (5), respectively, the rotatable handle (4) in a first position allowing an insertion of the wedge-shaped needle guide (5) and in a second position serving for fixing said needle guide (5) as well as allowing the needle (7) to penetrate said needle guide (5), and in a third position not allowing a needle (7) to penetrate said needle guide (5). In this way, a needle guide supporting device is obtained which is easier to operate by a physician during surgery.

## Description

The invention relates to a needle guide supporting device with clip means for detachable fastening to an ultrasound head, said device being shaped for detachable reception of a preferably wedge-shaped needle guide.

WO 96/10958 discloses a needle guide supporting device for use when performing for example liver biopsies on a patient. This device can be clipped on to an ultrasound head. The ultrasound head provides an image of the needle in relation to the internal organs. However, a disadvantage of this device is that it is unhandy during surgery.

The purpose of the invention is therefore to provide a needle guide supporting device which is easier to operate by a physician during surgery.

A needle guide supporting device of the above type is according to the invention characterised by being provided with a rotatable handle being rotatably adjustable and optionally fixable in various positions for the insertion and fastening of the needle guide, respectively. In this way, a needle guide supporting device is obtained which is easier to handle than hitherto known due to the rotatable handle.

Furthermore, according to the invention, the rotatable handle may in a first position allow an insertion of the wedge-shaped needle guide.

In addition, according to the invention, the rotatable handle may in a second position be suited for fixing the wedge-shaped needle guide while allowing a needle to penetrate the needle guide.

Finally, according to the invention, the rotatable handle may in a third position block a needle from penetrating the needle guide.

The invention is explained in detail below with reference to the drawings, in which
Fig. 1 shows a device according to the invention for supporting a wedge-shaped needle guide,
Fig. 2 shows the wedge-shaped needle guide attached to the device by means of a rotatable handle in a third position, the lower folded portion of the handle preventing a needle from penetrating the needle guide in said position,

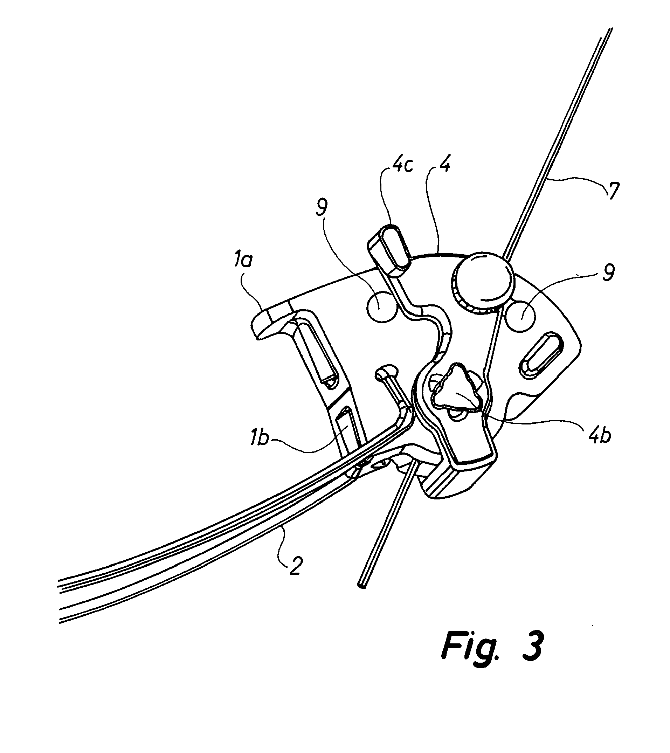

Fig. 3 shows the handle in a second position, the lower portion no longer preventing a needle from penetrating the needle guide in said position while fixing the needle guide,
Fig. 4 shows the needle guide supporting device detached from the needle guide,
Fig. 5 shows the needle guide supporting device attached to an ultrasound head,
Fig. 6 is a bottom view of the needle guide supporting device attached to the ultrasound head,
Fig. 7 is an alternative embodiment of the needle guide without grooves, and
Fig. 8 is an illustration of how to detach the handle from the needle guide supporting device.

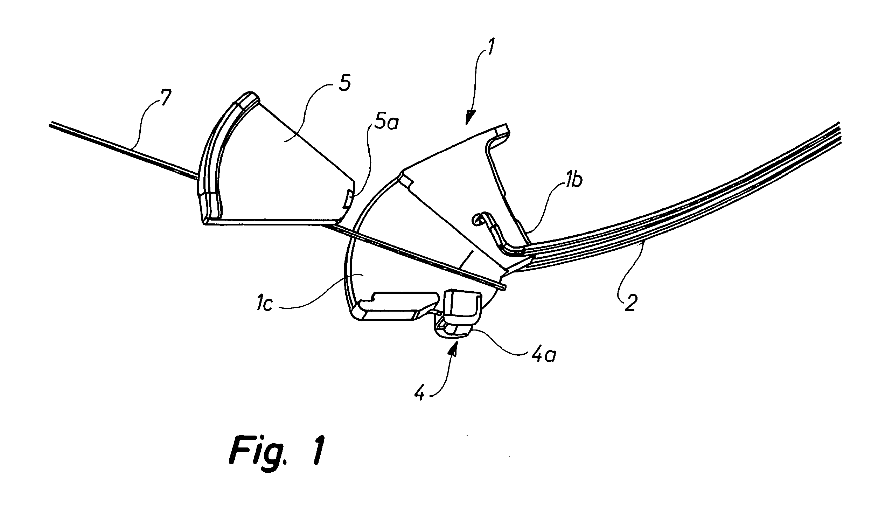

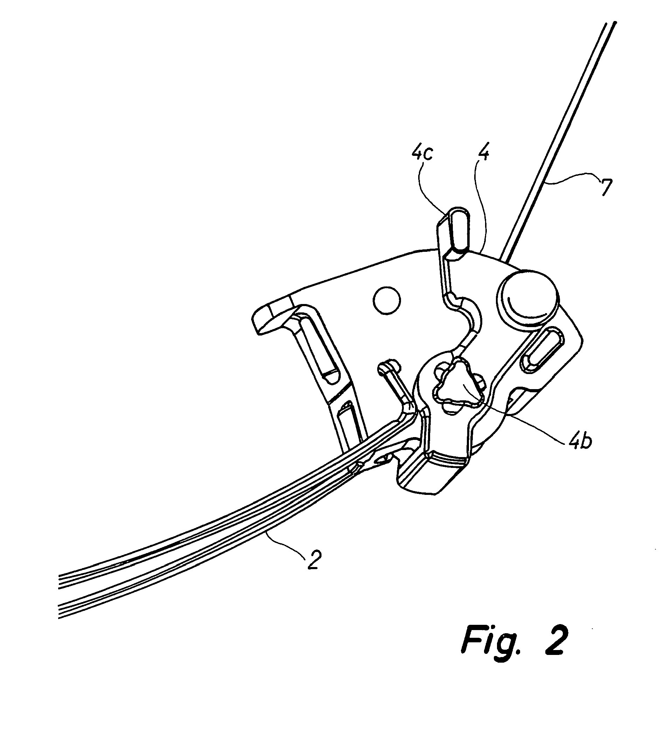

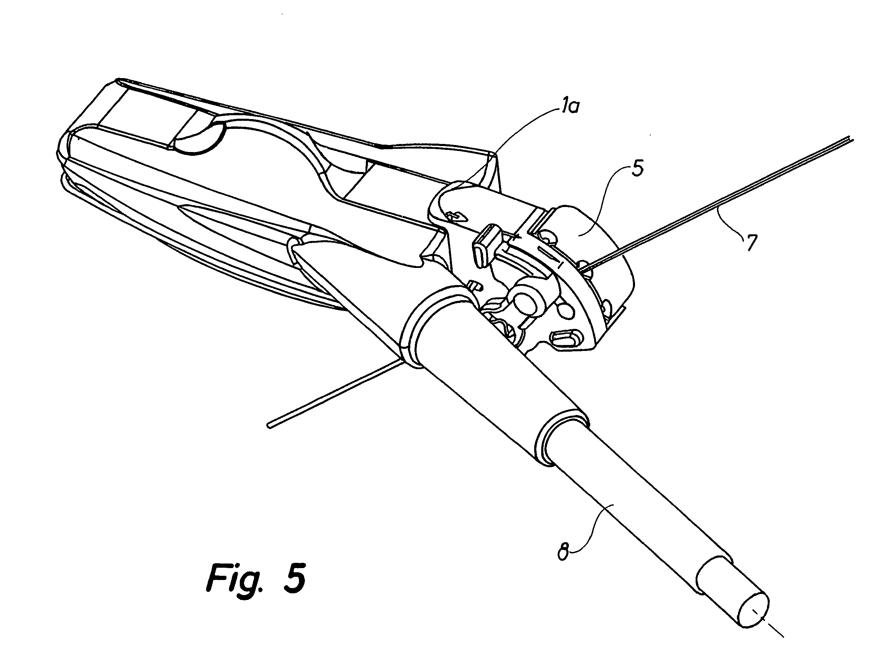

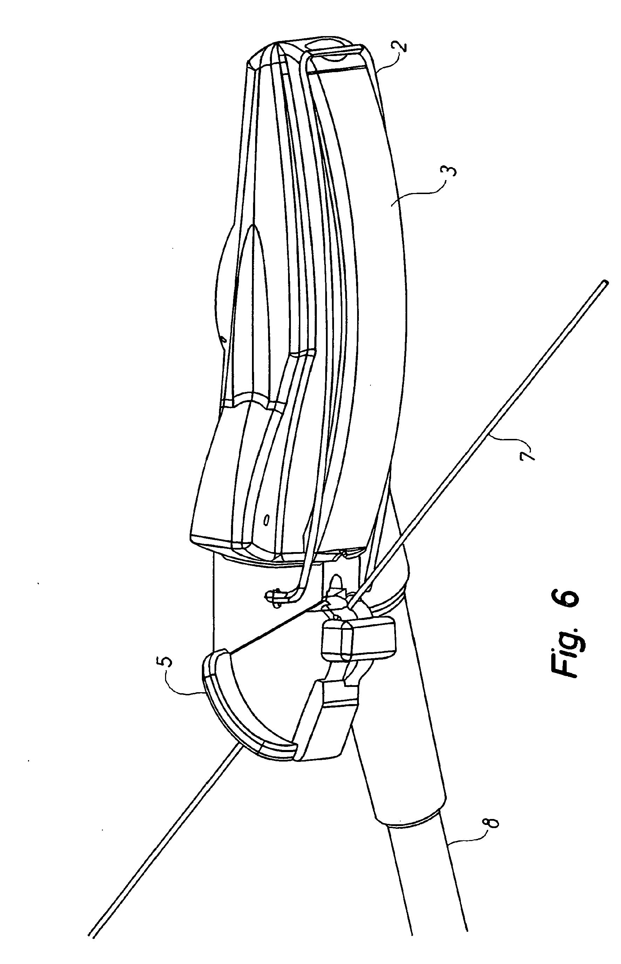

The needle guide supporting device 1 shown in Fig. 1 is provided with clip means 2 for fastening to a an ultrasound head 3 shaped as a circular arc as well as a rotatable handle 4 for fixing a needle guide 5. The device 1 is attached to the ultrasound head 3 in such a manner as shown in Figs. 5 and 6, the top of the device having a projecting member 1a which is moved in above the case of the ultrasound head, and below said projecting member, a projecting bulb 1b engaging a corresponding recess in the case is positioned. After arranging the device on the ultrasound head 3, the clip means 2 is moved up below the ultrasound array, shaped as a circular arc, of the ultrasound head in order to attach the device 1 to the ultrasound head. A wedge-shaped needle guide 5 can then be inserted in a corresponding wedge-shaped recess 1 c in the device. The rotatable handle 4 is then rotated into a second position, thereby fixing the wedge-shaped needle guide 5 due to a projecting pin 5a on the needle guide 5 and the folding 4a at the bottom of the handle 4, said folding engaging the pin 5a - cf. Fig. 2. When the handle is in this position, a needle 7 can penetrate the needle guide 5.

This will only be possible until the handle 4 is rotated all the way into a subsequent position. The rotatable handle 4, being rotatable about the centre of rotation 4b, is fixated in each of the aforementioned positions by means of a projecting spring pre-stressed ball in the handle 4, which can be inserted in corresponding recesses 9 in the device. The rotatable handle 4 is provided with a projecting grip 4c facilitating operation thereof.

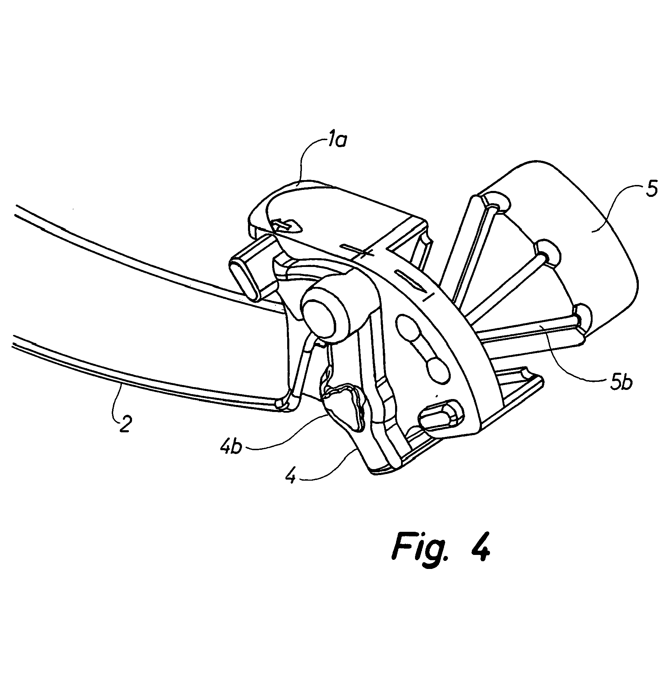

The inner side of the needle guide 5 is provided with a plurality of grooves 5b, each groove having an individual angle position, so that the physician is free to choose the angle position best suitable for the conditions when taking samples from internal organs of a patient. It appears from Fig. 4 that three grooves 5a are provided, each with their own angle position. Several different needle guides can be arranged for needles of different thickness.

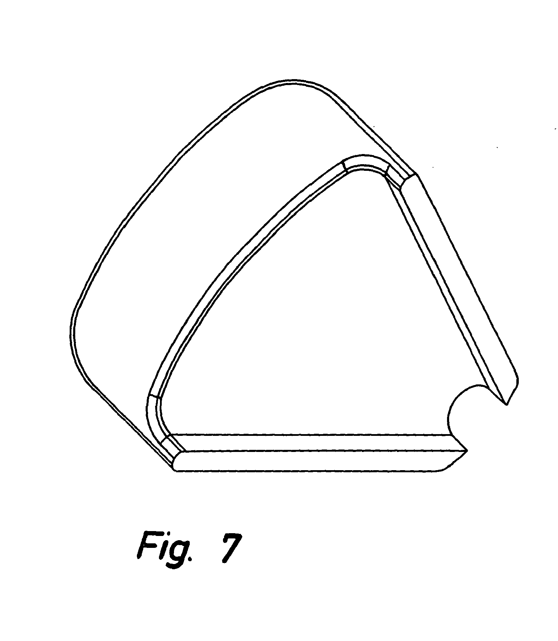

In an alternative embodiment of the needle guide - cf. Fig. 7 - there are no grooves, thereby enabling the physician to freely choose which angle the needle is to be inserted in the patient.

The ultrasound head 3 *per se* including an array of ultrasound components communicates via a cable 8 with a signal processing unit having a monitor, for example a signal processing unit B-K Medical of the type 2202 Pro Focus. The programme for processing the electrical signals from the ultrasound components is conventional.

The device according to the invention is suitable for taking samples from internal organs such as the liver of a patient diagnosed with liver cancer.

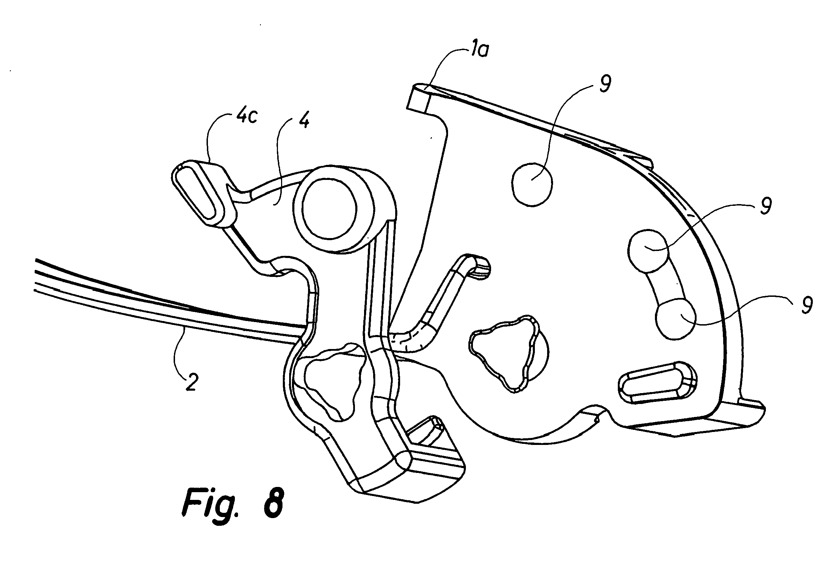

A particular advantage of the device according to the invention is that it is easy to disassemble and to disinfect, as the handle 4 can be rotated all the way back so that it can be detached - cf. Fig. 8.

## Claims

1. A needle guide supporting device with clips means (2) for detachable fastening to an ultrasound head (3), said device being shaped for detachable reception of a preferably wedge-shaped needle guide (5), **characterised in that** said device is provided with a rotatable handle (4) being rotatably adjustable and optionally fixable in various positions for the insertion and fastening of the needle guide (5), respectively.

2. A device according to claim 1, **characterised in that** the rotatable handle (4) in a first position allows an insertion of the wedge-shaped needle guide (5).

3. A device according to claim 1, **characterised in that** the rotatable handle (4) in a second position is suited for fixing the wedge-shaped needle guide (5) and allows the needle (7) to penetrate the needle guide (5).

4. A device according to claim 1, **characterised in that** the rotatable handle (4) in a third position blocks a needle (7) from penetrating the needle guide (5).

5. A device according to claim 1, **characterised in that** the rotatable handle is fixed in the various positions by means of a spring pre-stressed ball engaging corresponding recesses in said device.

6. A device according to claim 5, **characterised in that** it is ensured that the rotatable handle only can be detached when said handle is in a pre-determined angle position in relation to said device.

7. A device according to claim 6, **characterised in that** the handle only can be detached when the device is not attached to the ultrasound head.
